# EUROPÄISCHE PATENTSCHRIFT

(11) **EP 1 275 637 B1**
(45) Veröffentlichungstag und Bekanntmachung des Hinweises auf die Patenterteilung: **17.01.2007**
(21) Anmeldenummer: 02013987.9
(22) Anmeldetag: 26.06.2002
(51) Int. Cl.: C07C 253/14

(54) **Verfahren zur Herstellung von aromatischen Nitrilen**
Process for the preparation of aromatic nitriles
Procédé de préparation de nitriles aromatiques

(30) Priorität: 09.07.2001 DE 10133274
(43) Veröffentlichungstag der Anmeldung: 15.01.2003
(73) Patentinhaber: Saltigo GmbH, 51369 Leverkusen (DE)
(72) Erfinder: Eckert, Markus, Dr., 50937 Köln (DE)
(74) Vertreter: Wichmann, Birgid

(56) Entgegenhaltungen:
- DE-A- 10 015 280
- US-A- 4 211 721
- OKANO T. ET AL.: "CATALYTIC CYANATION OF ARYL HALIDES WITH NACN IN THE PRESENCE OF CROWNED PHOSPHINE COMPLEXES OF PALLADIUM UNDER SOLID-LIQUID TWO-PHASE CONDITIONS" SYNLETT, 1998, Seiten 243-244, XP002219573

## Beschreibung

Die Erfindung betrifft ein Verfahren zur Herstellung von aromatischen Nitrilen durch Umsetzung von Halogenaromaten oder Aryl-perfluorsulfonaten mit Alkalimetallcyaniden in Gegenwart von Palladium-Katalysatoren, Polyethern und Zink.

Die Palladium-katalysierte Substitution von Halogenen in aromatischen Verbindungen durch Alkalimetallcyanide ist zum Beispiel aus US-A 4,211,721 oder T. Okano et al., Synlett, 1998, S. 243 bekannt. Die dort beschriebenen Verfahren, besitzen allerdings den Nachteil, dass sowohl die Ausbeuten als auch die geringen Umsatzzahlen der Katalysatoren (Turnover-Number, TON) von maximal 170 die technische Realisierung unwirtschaftlich machen. Ein verbessertes Verfahren wurde von Maligres et al. in Tetrahedron Letters, 40, 1999, S. 8193 vorgestellt. Allerdings wird hier als Cyanidquelle das teure Zinkcyanid verwendet, was für den industriellen Maßstab nicht akzeptabel ist. Es bestand daher das Bedürfnis, ein Verfahren zu entwickeln, dass die Palladium-katalysierte Cyanierung von Halogenaromaten oder Aryl-perfluorsulfonaten unter Verwendung von billigen Alkalimetallcyaniden mit hohen Umsatzzahlen und Ausbeuten ermöglicht.

Es wurde nun ein Verfahren zur Herstellung von aromatischen Nitrilen der allgemeinen Formel (I),

Ar-[CN]ₙ (I),

in der
- Ar: für einen substituierten oder unsubstituierten aromatischen Rest und
- n: für eins oder zwei steht,
gefunden, dadurch gekennzeichnet, dass
a) eine aromatische Verbindung der allgemeinen Formel (II),

   Ar-[X]ₙ (II),

   in der
   - Ar und n: die oben genannte Bedeutung besitzen,
   und
   - X: jeweils unabhängig voneinander für Chlor, Brom, Iod oder einen Perfluoralkylsulfonyloxy-Rest stehen können
b) in Gegenwart eines Palladium-Katalysators und
c) Zink und
d) einem oder mehreren Polyethern
e) mit einem oder mehreren Alkalimetallcyaniden
f) gegebenenfalls unter Zusatz eines aprotischen Lösungsmittels umgesetzt wird.

Substituierte oder unsubstituierte aromatische Reste bedeuten in diesem Zusammenhang beispielsweise carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Fluor, Nitro, Cyano, Amino, Di(C₁-C₆-alkyl)-amino, (C₁-C₆-Alkyl)-amino, geschütztes oder freies Formyl, C₁-C₁₂-Alkyl, C₆-C₁₂-Aryl, C₇-C₁₃-Arylalkyl, C₁-C₁₂-Halogenalkyl, C₁-C₁₂-Halogenalkoxy, C₁-C₆-Alkylsulfonyl, (C₆-C₁₂)-Arylsulfonyl, (C₇-C₁₃)-Alkylarylsulfonyl C₁-C₁₂-Acyl, -CO-(C₆-C₁₂)-Aryl, C₁-C₁₄-Alkoxycarbonyl, -OCO-(C₆-C₁₂)-Aryl, -OCO-(C₇-C₁₃)-Arylalkyl, -NHCO-(C₁-C₈)-Alkyl, N(C₁-C₈-Alkyl)CO-(C₁-C₈)-Alkyl, -NHCO-(C₆-C₁₂)-Aryl, -NHCO-(C₇-C₁₃)-Arylalkyl, -COO-(C₁-C₁₂)-Alkyl, -CONH₂, -CON(C₁-C₆-Alkyl)₂, -CONH(C₁-C₆-Alkyl), -NCOO-(C₁-C₈)-Alkyl, -PO-[(C₁-C₈)-Alkyl]₂, -PO-[(C₆-C₁₂)-Aryl]₂, COO-(C₆-C₁₂)-Aryl, Aryloxy mit 6 bis 10 Gerüstkohlenstoffatomen, Heteroaryloxy mit 5 bis 12 Gerüstkohlenstoffatomen von denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können oder Resten der allgemeinen Formel (III),

(C₁-C₈-Alkyl)-A-B (III)

in der
- A: beispielsweise für Funktionalitäten ausgewählt aus der Gruppe 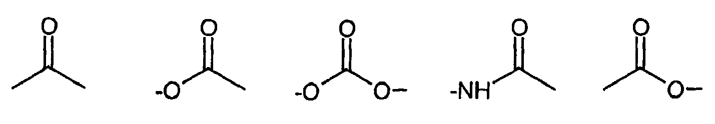 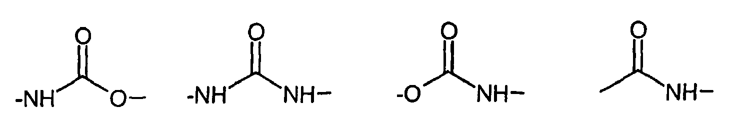
und
- B: für C₁-C₈-Alkyl, C₆-C₁₂-Aryl oder C₇-C₁₃-Arylalkyl oder
A und B zusammen für Cyano stehen können.

Alkyl bedeutet in den oben genannten Zusammenhängen jeweils unabhängig einen geradkettigen, cyclischen, verzweigten oder unverzweigten Alkyl-Rest.

Alkoxy bedeutet in den oben genannten Zusammenhängen jeweils unabhängig einen geradkettigen, cyclischen, verzweigten oder unverzweigten Alkoxy-Rest.

Halogenalkyl und Halogenalkoxy bedeuten in den oben genannten Zusammenhängen jeweils unabhängig geradkettige, cyclische, verzweigte oder unverzweigte Alkylreste und Alkoxyreste, die mit einem, mehreren oder vollständig mit Fluor- oder Chloratomen substituiert sein können.

Aryl bedeutet in den oben genannten Zusammenhängen jeweils unabhängig einen substituierten oder unsubstituierten aromatischen oder heteroaromatischen Rest.

Beispiele für carbocyclische aromatische Reste mit 6 bis 24 Gerüstkohlenstoffatomen sind zum Beispiel Phenyl, Naphtyl, Biphenyl, Binaphtyl oder Anthracenyl, heteroaromatische Reste mit 5 bis 24 Gerüstkohlenstoffatomen in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können sind beispielsweise Pyrimidinyl, Pyridinyl, Oxazolyl, Thiophenyl, Furanyl, Indolyl, Triazolyl oder Chinolinyl.

Bevorzugt werden für das erfindungsgemäße Verfahren aromatische Verbindungen der allgemeinen Formel (II) eingesetzt, in der

Ar für einen substituierten oder unsubstituierten Rest aus der Gruppe Phenyl, Naphtyl, Binaphtyl, Biphenyl, Pyrimidinyl. Oxazolyl oder Pyridinyl steht, die mit keinem, einem, zwei oder drei Resten pro Cyclus weiter substituiert sein können, die jeweils unabhängig voneinander ausgewählt sind aus der Gruppe

Fluor, Nitro, Cyano, Di(C₁-C₆-alkyl)-amino, C₁-C₄-Alkyl, C₆-C₁₂-Aryl, C₁-C₁₂-Fluoralkyl, C₁-C₆-Fluoralkoxy, C₁-C₄-Acyl, COO-(C₁-C₆)-Alkyl, -CON(C₁-C₆-Alkyl)_{2,} und n = 1 ist und X für Chlor, Brom, Iod, Trifluormethansulfonyloxy oder Nonafluorbutansulfonyloxy steht.

Besonders bevorzugt werden für das erfindungsgemäße Verfahren aromatische Verbindungen der allgemeinen Formel (II) eingesetzt, in der
Ar für einen Phenyl-Rest steht, der mit keinem, einem, zwei oder drei Resten weiter substituiert sein kann, die jeweils voneinander unabhängig ausgewählt sind aus der Gruppe
Fluor, Cyano, C₁-C₄-Alkyl, Trifluormethyl, Trifluormethoxy, Acetyl, COO-(C₁-C₆)-Alkyl, -CON(C₁-C₆-Alkyl)₂ und n = 1 ist und X für Chlor oder Brom steht.

Für das erfindungsgemäße Verfahren werden als Palladium-Katalysatoren Palladium-Phosphin-Komplexe eingesetzt, die entweder in situ aus Palladiumverbindungen und Diphosphin-Liganden hergestellt oder als isolierte Verbindungen eingesetzt werden, bevorzugt ist die in situ Herstellung aus einem oder mehreren Palladiumverbindungen und einem oder mehreren Diphosphin-Liganden.

Als isolierte Palladium-Phosphin-Komplexe werden solche der allgemeinen Formel (IV) eingesetzt,

[PdL₂An₂] (IV)

in der
- L₂: zusammen für ein Diphosphin und
- An: für das Anion einer Säure steht.

Bevorzugt steht in der allgemeinen Formel (IV) L₂ für Diphosphine der allgemeinen Formel (V),

(R¹)₂P-A-P(R¹)₂ (V)

in der
die Reste R¹ unabhängig voneinander für geradkettiges oder cyclisches, verzweigtes oder unverzweigtes C₁-C₈-Alkyl oder für durch R² und R³ substituiertes Phenyl- oder durch R² und R³ substituiertes Naphtyl- oder durch R² und R³ substituiertes Heteroaryl mit 5 bis 12 Gerüstkohlenstoffatomen stehen, wobei in diesem
R² und R³ jeweils unabhängig voneinander für Wasserstoff, geradkettiges, verzweigtes oder cyclisches C₁-C₄-Alkyl, geradkettiges, verzweigtes oder cyclisches C₁-C₆-Alkoxy, Fluor- oder Cyano- und

- A: für einen unsubstituierten oder substituierten Rest aus der Gruppe C₁-C₄-Alkylen, 1,2-Phenyl, 1,2-Cyclohexyl, 1,1'-Ferrocenyl, 1,2-Ferrocenyl, 2,2'-(1,1'-Binaphtyl) und 1,1'-Biphenyl und
- An: für Chlorid, Bromid, Iodid oder Acetat steht.

Bei der in situ Herstellung von Palladium-Phosphin-Komplexen aus Palladiumsalzen und Diphosphin-Liganden bevorzugte Diphosphine sind solche der allgemeinen Formel (V),

(R¹)₂P-A-P(R¹)₂ (V)

in der
die Reste R¹ und A die oben angegebene Bedeutung besitzen.

Besonders bevorzugte Diphosphine sind Bis-(diphenylphosphino)propan oder 1,1'-Bis-diphenylphosphinoferrocen.

Ganz besonders bevorzugt ist 1,1'-Bis-diphenylphosphinoferrocen.

Als Palladiumverbindungen für die in situ Herstellung von Palladium-Phosphin-Komplexen werden bevorzugt

Pd₂(dibenzylidenaceton)₃

oder
solche der allgemeinen Formel (VIa) eingesetzt,

Pd(Y¹)₂ (VIa)

in der
- Y¹: für Chlorid, Bromid, Acetat, Methansulfonat oder Trifluormethansulfonat steht,
oder Palladiumverbindungen der allgemeinen Formel (VIb)

Pd(Y²)₂L₂ (VIb)

in der
- Y: für Chlorid, Bromid, Acetat, Methansulfonat, Trifluormethansulfonat, Tetrafluoroborat oder Hexafluorophosphat steht und
- L: jeweils für Acetontril, Benzonitril oder Benzylnitril steht, oder
- L₂: zusammen für 1,5-Cyclooctadien steht,
oder Palladiumverbindungen der allgemeinen Formel (VIc)

M₂[Pd(Y³)₄] (VIc),

wobei
- Y³: für Chlorid oder Bromid steht und
- M: für Lithium, Natrium, Kalium, Ammonium oder organisches Ammonium steht.

Besonders bevorzugt ist Palladiumacetat.

Das molare Verhältnis von Phosphor zu Palladium in der Reaktionsmischung kann beispielsweise 1 bis 10 betragen, 1,8 bis 2,5 ist bevorzugt.

Für das erfindungsgemäße Verfahren kann das molare Verhältnis von auszutauschendem Halogen oder Perfluorsulfonat zu Palladium beispielsweise 10 bis 10000 betragen, bevorzugt ist ein Verhältnis von 100 bis 2000.

Das erfindungsgemäß einzusetzende Zink kann beispielsweise in Form von Staub, Pulver oder Granalien vorliegen. Der Einsatz in Form von Zinkstaub ist bevorzugt.

Die eingesetzte Menge kann dabei das 1,0 bis 100-fache der molaren Menge der eingesetzten Palladiumverbindung sein, bevorzugt ist das 1,0 bis 10-fache.

Als Polyether werden im erfindungsgemäßen Verfahren bevorzugt solche eingesetzt, die eine molare Masse von 100 bis 5000 besitzen oder Mischungen solcher Polyether.

### Dies können beispielsweise sein:

Kronenether wie beispielsweise 18-Krone-6, Dibenzo-18-Krone-6, 12-Krone-4, Kryptanden wie zum Beispiel Kryptand[2.2.2], verzweigte oder unverzweigte Polyethylenglycolether wie beispielsweise Glyme, Diglyme oder Triglyme oder Gemische von Polyethylenglycolethem wie zum Beispiel PEG 200, PEG 300, PEG 400, PEG 600 oder PEG 1200. Solche Gemische sind kommerziell z.B. von der Firma Merck-Schuchardt erhältlich und über die mittlere Molmasse definiert.

Besonders bevorzugt sind PEG 200, PEG 300 und PEG 400.

Für das erfindungsgemäße Verfahren können die Polyether beispielsweise in einer Menge von 0,1 ml bis 100 ml pro Liter Reaktionsmischung eingesetzt werden. Bevorzugt ist eine Menge von 1 bis 10 g pro Liter Reaktionsmischung. Bei Einsatz von Polyethern mit einer Molmasse unter 250 wie beispielsweise Glyme oder Diglyme können diese allerdings auch als alleiniges Lösungsmittel fungieren. In diesem Fall kann die Menge beispielsweise so gewählt werden, dass pro Mol Halogenaromat oder Arylperfluorsulfonat 50 bis 1000 ml, bevorzugt 100 bis 500 ml Polyether eingesetzt werden.

Als Alkalimetallcyanide können beispielsweise Lithiumcyanid, Natriumcyanid, Kaliumcyanid eingesetzt werden, bevorzugt sind Natriumcyanid und Kalimcyanid.

Für das erfindungsgemäße Verfahren werden beispielsweise 0,95 bis 1,5 mol, bevorzugt 1,0 bis 1,2 mol Alkalimetallcyanid pro auszutauschendes Halogen oder Perfluorsulfonat eingesetzt.

Gegebenenfalls wird erfindungsgemäßes Verfahren in Gegenwart eines oder mehrerer aprotischer Lösungsmittel durchgeführt. Bevorzugt sind dies:

Cyclische oder acyclische Ether wie 1,4-Dioxan, Tetrahydrofuran, Diethylether, oder, Methyl-tert.-butylether oder Di-n-butylether, aromatische Kohlenwasserstoffe wie zum Beispiel Toluol, o-Xylol, m-Xylol oder p-Xylol, dipolar aprotische Verbindungen wie zum Beispiel Dimethylformamid, N-Methylpyrolidon oder N-Methylcaprolactam oder Mischungen solcher Lösungsmittel.

Die Menge des gegebenenfalls eingesetzten aprotischen Lösungsmittels kann beispielsweise 50 ml bis 5000 ml bevorzugt 100 bis 500 ml pro Mol des eingesetzten Halogenaromaten oder des Arylperfluorsulfonats betragen.

Die Reaktionstemperatur beträgt zum Beispiel 50 bis 170°C, bevorzugt 100 bis 140°C.

Die Reaktion kann beispielsweise bei 0,2 bis 100 bar durchgeführt werden, bevorzugt ist Normaldruck.

Die Reaktionsdauer kann beispielsweise 2h bis 72 Stunden betragen, 12 bis 24 h sind bevorzugt.

Die Reaktion wird bevorzugt unter Schutzgasatmosphäre unter weitgehendem Ausschluss von Sauerstoff und Feuchtigkeit durchgeführt. Als Schutzgase kommen beispielsweise Stickstoff und Edelgase wie beispielsweise Argon oder Mischungen solcher Gase in Frage.

In einer bevorzugten Ausführungsform legt man den Halogenaromaten oder das Arylperfluorsulfonat zusammen mit der Palladiumverbindung, dem Diphosphin, dem Zink, dem Polyether und gegebenenfalls dem aprotischen Lösungsmittel vor, lässt zwischen 3 und 120 Minuten zur Präformierung der Palladium-Phosphin-Komplexe bei einer Temperatur von 50 bis 140°C rühren und gibt gegebenenfalls nach zwischenzeitlichem Abkühlen auf unter 50°C das Alkalimetallcyanid zu. Diese Vorgehensweise hat den Vorteil, dass eine Desaktivierung der Palladiumverbindung zum Beispiel durch Bildung von Cyanopalladaten weitestgehend vermieden wird.

Der Vorteil des erfindungsgemäßen Verfahrens liegt in der leichten Durchführbarkeit und den hohen Ausbeuten an aromatischen Nitrilen. Weiterhin werden hohe Katalysatorumsatzzahlen (TON) von über 500 Mol Halogenaromat/Mol Palladium-Katalysator erreicht. Das erfindungsgemäße Verfahren zeichnet sich insbesondere durch die Verwendung der billigen Alkalimetallcyanide als Cyanidquelle aus.

### Beispiele

### Beispiel 1

Unter Argon wurden 23,2 g 3-Brombenzotrifluorid (97 %ig), 0,023 g Palladiumacetat, 0,11 g 1,1-Bis-(diphenylphosphino)-ferrocen und 0,3 g PEG 400 sowie 15 ml trockenes Toluol vorgelegt und 30 Minuten unter Rühren zum Rückfluss erhitzt. Es wurde abgekühlt (<50°C) und 5,15 g gemahlenes Natriumcyanid sowie 0,078 g Zinkstaub hinzugegeben und die Mischung unter Rühren für 24 Stunden unter Rückfluss erhitzt. Nach dem Abkühlen wurde filtriert und die Lösung mittels GC analysiert (Gewichtsprozent): es wurden 10,6 g 3-Trifluormethylbenzonitril gefunden (62 % d.Th.).

### Beispiel 2

Wie in Beispiel 1, jedoch wurden 26,21 g 5-Brom-2-chlorbenzotrifluorid (99%ig) umgesetzt. Es wurden 19,1 g 4-Chlor-3-trifluormethyl-benzonitril gefunden (GC-Analytik: 93 % d. Th.).

### Beispiel 3

Wie Beispiel 1, jedoch wurde die Reaktion bei 135°C in 15 ml entgastem Xylol mit 0,3 g PEG 200 anstatt PEG 400 durchgeführt. Zur Aufarbeitung wurde die organische Phase dreimal mit Wasser gewaschen und anschließend im Vakuum fraktioniert destilliert. Es wurden 15,6 g 3-Trifluormethylbenzonitril als farblose Flüssigkeit isoliert (91 % d. Th.).

### Beispiel 4

Wie Beispiel 2, jedoch wurden als Ligand 0,082 g 1,3-Bis(diphenylphosphino)-propan eingesetzt. Es wurden 18,3 g 4-Chlor-3-trifluormethyl-benzonitril gefunden (GC-Analytik: 89 % d.Th.).

### Beispiel 5

Wie Beispiel 3, jedoch wurden 17,5 g 4-Bromfluorbenzol umgesetzt. Es wurden 10,1 g 4-Fluorbenzonitril gefunden (GC-Analytik: 83 % d.Th.).

### Beispiel 6

Wie Beispiel 2, jedoch wurden 0,012 g Palladiumacetat und 0,55 g 1,1-Bis-(diphenylphosphino)-ferrocen eingesetzt. Es wurden 12,5 g 4-Chlor-3-trifluormethylbenzonitril gefunden (GC-Analytik: 61 % d.Th.).

### Beispiel 7

Unter Argon wurden 26,21 g 5-Brom-2-chlorbenzotrifluorid (99%ig), 0,046 g Palladiumacetat, 0,22 g 1,1-Bis-(diphenylphosphino)-ferrocen und 0,3 g PEG 400 sowie 20 ml trockenes Dioxan vorgelegt und 30 Minuten unter Rühren zum Rückfluss erhitzt. Es wurde unter 50°C abgekühlt und 15,45 g gemahlenes Natriumcyanaid sowie 0,15 g Zinkstaub hinzugegeben und die Mischung unter Rühren für 16 Stunden unter Rückfuß erhitzt. Nach dem Abkühlen wurde filtriert und die Lösung mittels GC analysiert (Gewichtsprozent): es wurden 16,9 g 4-Cyano-3-trifluormethylbenzonitril gefunden (86 % d.Th.).

### Beispiel 8

Wir Beispiel 3, jedoch wurden 24,9 g 3,5-(Bistrifluormethyl)-chlorbenzol umgesetzt. Es wurden 15,5 g 3,5-(Bistrifluormethyl)-benzonitril als farblose Flüssigkeit isoliert (65 % d.Th.).

## Patentansprüche

1. Verfahren zur Herstellung von aromatischen Nitrilen der allgemeinen Formel (I),
Ar-[CN]ₙ (I),
in der
Ar für einen substituierten oder unsubstituierten aromatischen Rest und
n für eins oder zwei steht,
wobei
a) eine aromatische Verbindung der allgemeinen Formel (II),
Ar-[X]ₙ (II),
in der
Ar und n die oben genannte Bedeutung besitzen,
und
X jeweils unabhängig voneinander für Chlor, Brom, Iod oder einen Perfluoralkylsulfonyloxy-Rest stehen können,
b) in Gegenwart eines Palladium-Katalysators und
c) Zink und
d) einem oder mehreren Polyethern
e) mit einem oder mehreren Alkalimetallcyaniden umgesetzt wird, **dadurch gekennzeichnet, dass** als Palladium-Katalysator ein isolierter Palladium-Phosphin-Komplex der allgemeinen Formel (IV) eingesetzt wird,
[PdL₂An₂] (IV)
in der
L₂ zusammen für ein Diphosphin und
An für das Anion einer Säure steht
oder der Palladium-Phosphin-Katalysator in situ aus einer Palladiumverbindung und einem Diphosphin als Phosphin-Liganden erzeugt wird.

2. Verfahren nach Anspruch 1, **dadurch gekennzeichnet, dass** ein aprotisches Lösungsmittel zugesetzt wird.

3. Verfahren nach einem oder mehreren der Ansprüche 1 bis 2, **dadurch gekennzeichnet, dass** aromatische Verbindungen der allgemeinen Formel (II) eingesetzt werden, in der Ar für einen substituierten oder unsubstituierten Rest aus der Gruppe Phenyl, Naphtyl, Binaphtyl, Biphenyl, Pyrimidinyl, Oxazolyl und Pyridinyl steht, der mit keinem, einem, zwei oder drei Resten pro Cyclus weiter substituiert sein kann, die jeweils unabhängig voneinander ausgewählt sind aus der Gruppe Fluor, Nitro, Cyano, Di(C₁-C₆-alkyl)-amino, C₁-C₄-Alkyl, C₆-C₁₂-Aryl, C₁-C₁₂-Fluoralkyl, C₁-C₆-Fluoralkoxy, C₁-C₄-Acyl, COO-(C₁-C₆)-Alkyl, -CON(C₁-C₆-Alkyl)₂,
und n = 1 ist
und X für Chlor, Brom, Iod, Trifluormethansulfonyloxy oder Nonafluorbutansulfonyloxy steht.

4. Verfahren nach einem oder mehreren der Ansprüche 1 bis 3, **dadurch gekennzeichnet, dass** die Zugabe des Alkalimetallcyanids nach der Präformierung des Palladium-Phosphin-Komplexes erfolgt.

5. Verfahren nach einem oder mehreren der Ansprüche 1 bis 4, **dadurch gekennzeichnet, dass** die eingesetzten Polyether eine Molmasse von 100 bis 5000 besitzen.

6. Verfahren nach einem oder mehreren der Ansprüche 1 bis 5, **dadurch gekennzeichnet, dass** als Alkalimetallcyanid Natriumcyanid oder Kaliumcyanid eingesetzt wird.

7. Verfahren nach einem oder mehreren der Ansprüche 1 bis 6, **dadurch gekennzeichnet, dass** die Reaktion bei einer Temperatur von 50 bis 170°C durchgeführt wird.

8. Verfahren nach einem oder mehreren der Ansprüche 1 bis 7, **dadurch gekennzeichnet, dass** das Diphosphin ein solches der allgemeinen Formel (V) ist,
(R¹)₂P-A-P(R¹)₂ (V)
worin
die Reste R¹ unabhängig voneinander für geradkettiges oder cyclisches, verzweigtes oder unverzweigtes C₁-C₈-Akyl oder für durch R² und R³ substituiertes Phenyl- oder durch R² und R³ substituiertes Naphtyl- oder durch R² und R³ substituiertes Heteroaryl mit 5 bis 12 Gerüstkohlenstoffatomen stehen, wobei in diesem
R² und R³ jeweils unabhängig voneinander für Wasserstoff, geradkettiges, verzweigtes oder cyclisches C₁-C₄-Alkyl, geradkettiges, verzweigtes oder cyclisches C₁-C₆-Alkoxy, Fluor- oder Cyano- und
A für einen unsubstituierten oder substituierten Rest aus der Gruppe C₁-C₄-Alkylen, 1,2-Phenyl, 1,2-Cyclohexyl, 1,1'-Ferrocenyl, 1,2-Ferrocenyl, 2,2'-(1,1'-Binaphtyl) und 1,1'-Biphenyl steht.

9. Verfahren nach einem oder mehreren der Ansprüche 1 bis 8, **dadurch gekennzeichnet, dass** An für Chlorid, Bromid, Iodid oder Acetat steht.

## Claims

1. Process for the preparation of aromatic nitriles of the general formula (I),
Ar-[CN]ₙ (I),
in which
Ar represents a substituted or unsubstituted aromatic radical and
n represents one or two,
in which
a) an aromatic compound of the general formula (II),
Ar-[X]ₙ (II),
in which
Ar and n have the abovementioned meaning,
and
X in each case independently of one another can represent chlorine, bromine, iodine or a perfluoroalkylsulphonyloxy radical
is reacted
b) in the presence of a palladium catalyst and
c) zinc and
d) one or more polyethers
e) with one or more alkali metal cyanides.
**characterized in that** the palladium catalyst employed is an isolated palladium-phosphine complex of the general formula (IV),
[PdL₂An₂] (IV)
where
L₂ together represents a diphosphine and
An represents the anion of an acid,
or the palladium-phosphine catalyst is generated in situ from a palladium compound and a diphosphine as phosphine ligand.

2. Process according to Claim 1, **characterized in that** an aprotic solvent is added.

3. Process according to either or both of Claims 1 and 2, **characterized in that** aromatic compounds of the general formula (II) are employed in which Ar represents a substituted or unsubstituted radical from the group consisting of phenyl, naphthyl, binaphthyl, biphenyl, pyrimidinyl, oxazolyl and pyridinyl, which can be further substituted by no, one, two or three radicals per cycle, which in each case independently of one another are selected from the group consisting of fluorine, nitro, cyano, di(C₁-C₆-alkyl)-amino, C₁-C₄-alkyl, C₆-C₁₂-aryl, C₁-C₁₂-fluoralkyl, C₁-C₆-fluoralkoxy, C₁-C₄-acyl, COO-(C₁-C₆)-alkyl, -CON(C₁-C₆-alkyl)₂,
and n = 1
and X represents chlorine, bromine, iodine, trifluoromethanesulphonyloxy or nonafluorobutanesulphonyloxy.

4. Process according to one or more of Claims 1 to 3, **characterized in that** the addition of the alkali metal cyanide takes place after the preformation of the palladium-phosphine complex.

5. Process according to one or more of Claims 1 to 4, **characterized in that** the polyethers employed have a molar mass of 100 to 5000.

6. Process according to one or more of Claims 1 to 5, **characterized in that** the alkali metal cyanide employed is sodium cyanide or potassium cyanide.

7. Process according to one or more of Claims 1 to 6, **characterized in that** the reaction is carried out at a temperature of 50 to 170°C.

8. Process according to one or more of Claims 1 to 7, **characterized in that** the diphosphine is of the general formula (V),
(R¹)₂P-A-P(R¹)₂ (V)
where
the radicals R¹ independently of one another represent straight-chain or cyclic, branched or unbranched C₁-C₈-alkyl or phenyl which is substituted by R² and R³ or naphthyl which is substituted by R² and R³ or heteroaryl having 5 to 12 ring carbon atoms which is substituted by R² and R³, where, in this radical
R² and R³ in each case independently of one another represent hydrogen, straight-chain, branched or cyclic C₁-C₄-alkyl, straight-chain, branched or cyclic C₁-C₆-alkoxy, fluorine- or cyano- and
A represents an unsubstituted or substituted radical from the group consisting of C₁-C₄-alkylene, 1,2-phenyl, 1,2-cyclohexyl, 1,1'-ferrocenyl, 1,2-ferrocenyl, 2,2'-(1,1'-binaphthyl) and 1,1'-biphenyl.

9. Process according to one or more of Claims 1 to 8, **characterized in that** An represents chloride, bromide, iodide or acetate.

## Revendications

1. Procédé de préparation de nitriles aromatiques de formule générale (I),
Ar-[CN]ₙ (I),
dans laquelle
Ar représente un radical aromatique substitué ou non substitué, et
n vaut un ou deux,
dans lequel
a) un composé aromatique de formule générale (II),
Ar-[X]ₙ (II),
dans laquelle
Ar et n présentent la signification mentionnée ci-dessus,
et
X peut représenter, dans chaque cas indépendamment l'un de l'autre, un atome de chlore, un atome de brome, un atome d'iode ou un radical perfluoroalkylsulfonyloxy,
est mis à réagir
b) en présence d'un catalyseur au palladium, et
c) de zinc, et
d) d'un ou de plusieurs polyéthers,
e) avec un ou plusieurs cyanures de métaux alcalins,
**caractérisé en ce que** l'on utilise, en tant que catalyseur au palladium, un complexe de palladium-phosphine isolé de formule générale (IV),
[PdL₂An₂] (IV)
dans laquelle
L₂ représente conjointement une diphosphine, et
An représente l'anion d'un acide,
ou le catalyseur palladium-phosphine est généré in situ à partir du composé du palladium et d'une diphosphine en tant que ligand phosphine.

2. Procédé selon la revendication 1, **caractérisé en ce qu'**un solvant aprotique est ajouté.

3. Procédé selon l'une ou plusieurs des revendications 1 à 2, **caractérisé en ce que** des composés aromatiques de formule générale (II) sont utilisés, dans laquelle Ar représente un radical substitué ou non substitué parmi le groupe constitué d'un groupe phényle, d'un groupe naphtyle, d'un groupe binaphtyle, d'un groupe biphényle, d'un groupe pyrimidinyle, d'un groupe oxazolyle et d'un groupe pyridinyle, qui peut être en outre substitué par zéro, un, deux ou trois radicaux par cycle, qui sont choisis, dans chaque cas indépendamment les uns des autres, parmi le groupe constitué d'un atome de fluoré, d'un groupe nitro, d'un groupe cyano, d'un groupe di(C₁-C₆-alkyl)-amino, d'un groupe alkyle en C₁-C₄, d'un groupe aryle en C₆-C₁₂, d'un groupe fluoroalkyle en C₁-C₁₂, d'un groupe fluoroalcoxy en C₁-C₆, d'un groupe acyle en C₁-C₄, d'un groupe COO-(C₁-C₆)-alkyle, d'un groupe -CON(C₁-C₆-alkyle)₂,
et n vaut 1,
et X représente un atome de chlore, un atome de brome, un atome d'iode, un groupe trifluorométhanesulfonyloxy ou un groupe nonafluorobutanesulfonyloxy.

4. Procédé selon l'une ou plusieurs des revendications 1 à 3, **caractérisé en ce que** l'addition du cyanure de métal alcalin est réalisée après la préformation du complexe palladium-phosphine.

5. Procédé selon l'une ou plusieurs des revendications 1 à 4, **caractérisé en ce que** les polyéthers utilisés présentent une masse molaire de 100 à 5000.

6. Procédé selon l'une ou plusieurs des revendications 1 à 5, **caractérisé en ce que** l'on utilise du cyanure de sodium ou du cyanure de potassium en tant que cyanure de métal alcalin.

7. Procédé selon l'une ou plusieurs des revendications 1 à 6, **caractérisé en ce que** la réaction est effectuée à une température de 50 à 170°C.

8. Procédé selon l'une ou plusieurs des revendications 1 à 7, **caractérisé en ce que** la diphosphine est une diphosphine de formule générale (V),
(R¹)₂P-A-P(R¹)₂ (V)
dans laquelle
les radicaux R¹ représentent, indépendamment l'un de l'autre, un groupe alkyle en C₁-C₈ à chaîne linéaire ou cyclique, ramifié ou non ramifié, ou un groupe phényle substitué par R² et R³, un groupe naphtyle substitué par R² et R³, ou un groupe hétéroaryle substitué par R² et R³, renfermant de 5 à 12 atomes de carbone du squelette, où, dans celui-ci,
R² et R³ représentent, dans chaque cas indépendamment l'un de l'autre, un atome d'hydrogène, un groupe alkyle en C₁-C₄ à chaîne linéaire, ramifié ou cyclique, un groupe alcoxy en C₁-C₆ à chaîne linéaire, ramifié ou cyclique, un atome de fluore ou un groupe cyano, et
A représente un radical non substitué ou substitué parmi le groupe constitué d'un groupe alkylène en C₁-C₄, d'un groupe 1,2-phényle, d'un groupe 1,2-cyclohexyle, d'un groupe 1,1'-ferrocényle, d'un groupe 1,2-ferrocényle, d'un groupe 2,2'-(1,1'-binaphtyle) et d'un groupe 1,1'-biphényle.

9. Procédé selon l'une ou plusieurs des revendications 1 à 8, **caractérisé en ce que** An représente un groupe chlorure, un groupe bromure, un groupe iodure ou un groupe acétate.
